# EUROPÄISCHE PATENTANMELDUNG

(11) **EP 3 258 268 A1**
(43) Veröffentlichungstag der Anmeldung: **20.12.2017**
(21) Anmeldenummer: 16174672.2
(22) Anmeldetag: 15.06.2016
(51) Int. Cl.: G01N 33/564, C07K 17/00

(54) **MARKERSEQUENZEN ZUR THERAPIESTEUERUNG VON PATIENTEN MIT RHEUMATOIDER ARTHRITIS**

(71) Anmelder: Protagen AG, 44227 Dortmund (DE)
(72) Erfinder: LÜKING, Angelika, 44892 Bochum (DE); SCHULZ-KNAPPE, Peter, 30966 Hemmingen (DE); BUDDE, Petra, 44229 Dortmund (DE); ZUCHT, Hans-Dieter, 30539 Hannover (DE)
(74) Vertreter: Simandi, Claus

(57) **Zusammenfassung**

Die vorliegende Erfindung betrifft ein Verfahren zur Therapiesteuerung von Patienten und Patienten(sub)populationen (Responder/Non-Responder) mit rheumatoider Arthritis (RA) als auch die Verwendung von geeigneten Markersequenzen, insbesondere in Form von Paneln, Diagnostika und Test-Kits, sowie deren Verwendung und Anwendung zur Diagnose, Prognose und Therapiesteuerung von rheumatoider Arthritis (RA), insbesondere bei einer medikamentösen Behandlung.

## Beschreibung

Die vorliegende Erfindung betrifft ein Verfahren zur Therapiesteuerung von Patienten und Patienten(sub)populationen (Responder/Non-Responder) mit rheumatoider Arthritis (RA) als auch die Verwendung von geeigneten Markersequenzen, insbesondere in Form von Paneln, Diagnostika und Test-Kits, sowie deren Verwendung und Anwendung zur Diagnose, Prognose und Therapiesteuerung von rheumatoider Arthritis (RA), insbesondere bei einer medikamentösen Behandlung.

Die rheumatoide Arthritis (RA) ist eine Autoimmunerkrankung an der etwa 1% der Bevölkerung der westlichen Welt leiden. Chronisch-entzündliche Prozesse führen bereits im ersten Jahr der Erkrankung (frühe RA) zu einer fortschreitenden gelenkzerstörenden Synovitis. Wird die RA nicht rechtzeitig erkannt und in einem bestimmten Zeitfenster ("window of opportunity") aggressiv behandelt, dann führt dies zu einer Gelenkzerstörung mit erheblichen körperlichen Einschränkungen und systematischen Manifestationen, die zu Behinderungen und verkürzter Lebenserwartung führen.

In der Frühphase der Erkrankung ist für Patienten mit einer Gelenkentzündung die Kriterien zur Diagnose einer RA und eine Unterscheidung zwischen einer Arthritis und einer reinen Arthralgie (Gelenkbeschwerden) häufig schwierig. Dies verzögert die Therapiesteuerung für Patienten mit einer möglicherweise frühen RA, so dass bei einer ungeklärten Diagnose die Gelenkzerstörung voranschreiten kann.

Daher sind Marker zur Früherkennung der RA bzw. Prognose und Therapiesteuerung immens wichtig, insbesondere bei solchen Patienten der rheumatoiden Arthritis (RA), die bereits in medikamentöser Behandlung sind.

Die aktuellen Klassifikationskriterien, welche das American College for Rheumatology (ACR) und die European League Against Rheumatism (EULAR) in 2010 gemeinsam veröffentlicht haben (Aletaha, Neogi et al. 2010), sollen durch die Bestimmung von Autoantikörpern (AAB) gegen citrullinierte Antigene (ACPA), eine frühe Diagnose der RA erleichtern, so daß eine krankheitsmodifizierende Therapie möglichst früh eingeleitet wird und irreversible Krankheitsfolgen verhindert werden.

ACPA Autoantikörper sind in etwa 75% von Patienten mit etablierter RA positiv nachweisbar, jedoch nur in etwa 62% der Patienten mit früher RA.

Dies unterstreicht den Bedarf an weiteren Markern für die Frühdiagnose der RA, Prognose und Therapiesteuerung.

Markersequenzen zur Diagnose von RA sind in WO2009030226 offenbart. Diese Markersequenzen wurden durch ein Verfahren gefunden, bei dem Serumproben von RA Patienten und solche von Gesunden vergleichend untersucht und die Ergebnisse statistisch ausgewertet wurden. Die in WO 2009030226 beschriebenen Markersequenzen sind zur Diagnose von RA geeignet, jedoch nicht ausreichend für die Vorhersage der Therapieantwort geeignet.

Bisher gilt die RA als eine Erkrankung, wobei vorwiegend Autoantikörper gegen citrullinierte Peptide produziert werden. In einigen wenigen Publikationen wurden auch Autoantikörper gegen posttranslational unmodifizierte Proteine oder Peptide beschrieben (Hueber, Tomooka et al. 2009; Somers, Geusens et al. 2011), jedoch wurde nicht systematisch nach neuen Autoantikörpern gesucht oder diese für die Identifizierung von Patienten(sub)gruppen eingesetzt.

Auf Basis der 2010 veröffentlichten Empfehlungen der European League Against Rheumatism (EULAR)für die Behandlung von RA ist das primäre Behandlungsziel, die Krankheitsprogression zu stoppen und die Krankheitsremission zu erreichen (Smolen, Landewe et al. 2010).

Durch den Einsatz einer krankheitsmodifizierenden Therapie (DMARD = Disease Modifying Anti-Rheumatic Drug, so genannte Basistherapie), läßt sich insbesondere in der frühen RA, die Erfolgsaussicht einer Krankheitsremission signifikant verbessern.

Unter klassischen DMARDs wird eine Gruppe von Medikamenten zusammengefasst, die über symptomatische Effekte hinaus, krankheitsmodifizierende Eigenschaften besitzt. Die Wirkung der DMARDs setzt in der Regel verzögert ein, der Zeitraum bis zum Wirkeintritt beträgt 4-16 Wochen.

Insbesondere das zur Gruppe der klassichen DMARD gehörende Methotrexate (MTX) ist zur Standard- und Ersttherapie der RA geworden. Andere geeignete Arzneimittel sind Azathioprin, Sulfasalazin, Chloroquin/Hydroxychloroquin, Leflunomid, Cyclophosphamid, D-Penicillamin oder Ciclosporin als auch biologische DMARDs.

Zu den sogenannten biologischen DMARDs gehören therapeutische Antikörper, die die Wirkung des inflammatorischen Zytokins - Tumor Nekrose Faktor Alpha (TNF) - blockieren. TNF ist zentraler Regulator des Immunsystems und an entzündlichen Prozessen in einer Reihe von rheumatoiden Erkrankungen, wie z.B. RA, Lupus, ankylosierende Spondylitis, Psoriasis, Morbus Crohn, Colitis ulcerosa und Juvenile Chronische Arthritis beteiligt.

Die Entwicklung von sogenannten TNF-Blockern hat die Behandlung der RA und anderen rheumatischen Erkrankungen signifikant verbessert und zu einer deutlichen Verbesserung der Lebensqualität geführt.

Generell gibt es zwei Ansätze zur Hemmung der TNF Aktivität. Zum einen können mittels monoklonaler therapeutischer Antikörper, die Bindung von TNF an seinen Rezeptor unterbunden werden. Hierzu sind verschiedene anti-TNF Blocker für die Behandlung der RA zugelassen, wie z.B. Infliximab (Remicade®; Adalimumab (Humira®), Certolizumab Pegol (Cimzia®) und Golimumab (Simponi®).

Weiterhin besteht die Möglichkeit durch löslichen, rekombinant hergestellten TNF-Rezeptor, wie z.B. Etanercept (Enbrel®) die Bindung von TNF an den körpereigenen Rezeptor zu hemmen.

Trotz generell guter Wirksamkeit zeigen lediglich 40-50% der RA Patienten eine zufriedenstellende klinische Verbesserung unter MTX-Therapie. Weiterhin kann eine MTX-Therapie von toxischen Effekten und unerwünschten Nebenwirkungen begleitet sein, die oftmals dazu führen, dass Patienten eine MTX-Therapie abbrechen.

In zahlreichen klinischen Studien konnte gezeigt werden, dass die verschiedenen TNF-Blocker eine vergleichbare Wirksamkeit gegenüber MTX zeigen und die Progression der RA mit Schädigung der Gelenke weitgehend hemmen. Jedoch zeigen klinische Studien, dass etwa 20-50% der behandelten RA Patienten nur unzureichend auf TNF-Blocker ansprechen. Für diese Patienten wurden alternative Therapien entwickelt, die z.B. auf der Hemmung von B-Zellen basieren, wie z.B. Rituximab (Rituxan).

In Anbetracht der hohen Behandlungskosten und möglichen Nebenwirkungen, fehlt es jedoch an geeigneten Prädikatoren, also diagnostischen Markersequenzen für die Auswahl von bestimmten und geeigneten Therapieformen als auch klinische Entscheidungen für RA Patienten.

Durch die erfindungsgemäße Verwendung von Marker(sequenze)n zur Identifizierung von Patienten, die auf eine MTX-Therapie ansprechen (Responder = R) bzw. nicht-ansprechen (Non-Responder = NR), können für Patienten(sub)gruppen mit spezifischem Markerprofil, die für sie beste Therapie ausgewählt werden.

Entsprechend der EULAR- und ACR-Empfehlungen werden nach Diagnosestellung zunächst konventionelle DMARDs (zumeist MTX) verabreicht und die klinische Wirksamkeit über sechs Monate verfolgt. Bei fehlender Wirksamkeit oder Nebenwirkungen kann auf ein biologisches DMARD, zumeist ein TNF-Blocker gewechselt werden. Dies hat jedoch den Nachteil, dass bei fehlender Wirksamkeit wertvolle Zeit verstreicht und die Erkrankung ungehindert weiter fortschreitet.

Klinische Studien zeigen, dass ebenfalls eine frühzeitige intensive Behandlung von Patienten mit einer frühen RA mit einer Kombinationstherapie aus TNF-Blocker und MTX im Vergleich zu einer MTX-Monotherapie die Prognose der Erkrankung bessern kann (Detert, Bastian et al. 2013) und eine alternative medikamentöse Behandlung darstellt.

Allerdings fehlen geeignete Prädikatoren bzw.

Marker(sequenzen), um (Sub-)Gruppen von Patienten zu identifizieren, die von einer solchen aggressiven Kombinationstherapie profitieren können.

Daher ist es Aufgabe der vorliegenden Erfindung die Therapie mittels medikamentöser Behandlung (DMARD) als auch hinsichtlich klinischer Entscheidungen zu verbessern.

Die Aufgabe wird durch die technische Lehre der Patentansprüche gelöst.

Aufgabe der Erfindung ist daher ebenfalls Prognose und Therapiesteuerung mittels einer in-vitro Diagnose in der Behandlung von RA-Patienten. Insbesonderer solcher RA-Patienten, die bereits eine medikamentöse Behandlung erfahren.

Diese Aufgabe wird erfindungsgemäß dadurch gelöst, dass ein Verfahren zur in-vitro Diagnose bereitgestellt wird, welche ausgewählte Autoantigene (Marker) enthält zum Nachweis von Autoantikörpern aus Köperflüssigkeiten eines RA-Patienten, welcher eine medikamentöse Behandlung erfährt.

Daher betrifft die Erfindung ein Verfahren zur Stratifizierung und Therapiesteuerung von Patienten mit rheumatoider Arthritis in medikamentöser Behandlung, wobei mittels einer in-vitro Diagnose Responder und / oder Non-Responder auf die medikamentöse Behandlung identifiziert werden, wobei mindestens eine Markersequenz aus der Gruppe SEQ ID No. 1-208 ausgewählt ist.

Die erfindungsgemäßen Autoantigene bzw. Marker(sequenzen) werden idenifiziert mittels eines differentiellen, eine Vielzahl von Schritten umfassendes Verfahren, bei dem Serumproben von Gesunden und RA-Patienten in medikamentöser Behandlung vergleichend im Hinblick auf ihre Reaktivität mit einer Vielzahl von potentiellen Antigenen untersucht und diese Ergebnisse statistisch ausgewertet werden.

Die Auswahl der Serumproben und die Abfolge der Schritte ermöglicht in überraschender Weise die Identifizierung von Marker(sequenze)n in Patienten mit früher und etablierter RA in medikamentöser Behandlung, die auch geeignet sind, RA Patienten(sub)gruppen zu identifizieren und die Erfolgsaussichten auf einen Therapieerfolg erhöhen können.

Gegenstand der Erfindung ist daher ein Verfahren zur Identifizierung von Markersequenzen für Rheumatoide Arthritis (RA), umfassend die Schritte:
a) Serumproben von RA-Patienten werden mit mehr als 5.000 an (Luminex-)Beads gekoppelten Antigenen in Kontakt gebracht, die Bindung der einzelnen Antigene an Proteine im Serum der RA Patienten durch Immunfluoreszenzassay gemessen und die mittlere Fluoreszenzintensität (MFI = median fluorescence intensity) für jedes einzelne Antigen bestimmt;
b) Serumproben von Gesunden werden mit denselben an (Luminex-)Beads gekoppelten Antigenen in Kontakt gebracht, die Bindung der einzelnen Antigene an Proteine im Serum von Gesunden durch Immunfluoreszenzassay gemessen und daraus die mittlere Fluoreszenzintensität (MFI = median fluorescence intensity) für jedes einzelne Antigen bestimmt;
c) die MFI Daten jedes einzelnen Antigens aus a) und b) werden statistisch mittels univariater Analyse ausgewertet und dadurch Marker identifiziert, mit denen RA-Patienten von Gesunden differenziert werden können;
d) und wobei die Marker ausgewählt werden aus den Sequenzen SEQ ID No. 1 -208, Teilsequenzen oder Fragmente davon, Homologen der Sequenzen SEQ ID No. 1 -208 mit mindestens 90 % Homologie.

Im Bereich der Mikroarrays werden ebene Substrate verwendet, an die Markersequenzen oder zu untersuchende Sequenzen gebunden sind. In Proteinbiochips sind die zu untersuchenden Markersequenzen oder die an diese Markersequenzen bindenden Sequenzen auf einem festen, ebenen Träger immobilisiert. Eine alternative Anordnung oder Panel von Markersequenzen oder zu untersuchenden Sequenzen ist auf Beads möglich, die sich deshalb unter anderem hinsichtlich ihrer Sensitivität und Spezifität von herkömmlichen Mikroarrays unterscheiden. Bead Arrays werden beispielsweise durch Imprägnieren von Kügelchen entweder mit unterschiedlichen Konzentrationen an Fluoreszenzfarbstoff oder z.B. durch Strichcode-Technologie erstellt. Die Kügelchen sind adressierbar und können verwendet werden, um spezifische Bindungsereignisse, die auf ihrer Oberfläche auftreten, zu identifizieren. Grundlage der Bead-Technologie sind mikroskopisch kleine sphärische Kügelchen oder Plättchen, die Mikrosphären oder Beads genannt werden. Diese Beads können analog zu ELISA und Western Blot als Festphase für biochemische Nachweisreaktionen dienen. Es sind verschiedenste Bead-Typen verfügbar, die sich beispielsweise in ihrem Fluoreszenzfarbton unterscheiden und von denen jeder ein eigenes spezifisches Nachweisreagenz auf der Oberfläche trägt. Auf diese Weise können entsprechend viele verschiedene Nachweisreaktionen in einem sehr geringen Probenvolumen simultan durchgeführt werden. Mit Bead Arrays sind spezifische Interaktion zweier definierter biochemischer Verbindungen nachweisbar. Im Vergleich zu herkömmlichen Mikroarrays zeichnet sich die Bead-basierte Validierung durch eine besonders hohe Sensitivität und Spezifität aus. Mit dem erfindungsgemäßen Verfahren und der Verwendung von Beads zur Validierung können Markersequenzen für RA identifiziert werden, die sich hinsichtlich ihrer Sensitivität und Spezifität von den bisher bekannten Markersequenzen unterscheiden.

Gegenstand der Erfindung ist auch eine Markersequenz für Rheumatoide Arthritis erhältlich durch ein erfindungsgemäßes Verfahren und wobei die Markersequenz ausgewählt wird aus der Gruppe der Sequenzen SEQ ID No. 1-208, Teilsequenzen oder Fragmente davon, Homologen der Sequenzen SEQ ID No. 1-208 mit mindestens 90 % Homologie.

Gegenstand der Erfindung ist auch die Verwendung von einer oder mehreren erfindungsgemäßen Markersequenz(en) zur Stratifizierung und Therapiesteuerung von Rheumatoider Arthritis.

Eine weitere Ausführungsform betrifft die erfindungsgemäße Verwendung dadurch gekennzeichnet, dass 2 oder 3, vorzugsweise 4 oder 5, besonders bevorzugt 6, 7 oder 8 oder mehr verschiedene Markersequenzen, beispielsweise 10 bis 20 oder 30 oder mehr verschiedene Markersequenzen verwendet werden. Ein solches Set an Markersequenzen wird Panel genannt.

Eine Ausführungsform betrifft die erfindungsgemäße Verwendung dadurch gekennzeichnet, dass die Markersequenz(en) auf einen festen Träger aufgebracht wird/werden, wobei der feste Träger ausgewählt wird aus Filter, Membranen, Wafer, beispielsweise Silizium-Wafer, Glas, Metall, Kunststoff, Chips, massenspektrometrische Targets, Matrix, Beads, beispielsweise magnetische, beschichtete oder markierte Beads, wie Fluorophor-markierte Beads oder Luminex-Beads.

Gegenstand der Erfindung ist auch ein Verfahren zur Stratifizierung oder Therapiesteuerung von Rheumatoider Arthritis, wobei
a.) mindestens eine erfindungsgemäße Markersequenz aus SEQ ID No. 1-208 auf einen festen Träger, vorzugsweise auf ein Bead aufgebracht wird und
b.) mit Körperflüssigkeit oder Gewebeauszug eines Patienten in Kontakt gebracht wird und
c.) der Nachweis einer Wechselwirkung der Körperflüssigkeit oder des Gewebeauszugs mit der Markersequenz aus a.) erfolgt.

Der Nachweis einer solchen Wechselwirkung kann beispielsweise durch eine Sonde, insbesondere durch einen Antikörper erfolgen.

Gegenstand der Erfindung ist auch ein Verfahren zum Stratifizieren, insbesondere zur Risikostratifizierung, oder zur Therapiesteuerung eines Patienten mit Rheumatoider Arthritis, wobei mindestens eine erfindungsgemäße Markersequenz verwendet wird, um eine Probe des Patienten zu untersuchen.

Eine Ausführungsform betrifft ein erfindungsgemäßes Verfahren zur Diagnose von Rheumatoider Arthritis, wobei das Stratifizieren oder die Therapiesteuerung Entscheidungen zur Behandlung und Therapie des Patienten, insbesondere Hospitalisierung des Patienten, Einsatz, Wirkung und / oder Dosierung eines oder mehrerer Arzneimittel, eine therapeutische Maßnahme oder die Überwachung eines Krankheitsverlaufes sowie Therapieverlaufs, Ätiologie oder Klassifizierung einer Erkrankung samt Prognose umfasst.

Gegenstand der Erfindung ist ebenfalls eine Anordnung oder Panel umfassend oder bestehend aus einer oder mehreren erfindungsgemäßen Markersequenz(en). Bevorzugte Panel sind Gegenstand der Beispiele.

Gegenstand der Erfindung ist ebenfalls ein Assay oder Proteinarray umfassend eine erfindungsgemäße Anordnung oder Panel.

Gegenstand der Erfindung ist auch ein Diagnostikum zur Diagnose von Rheumatoider Arthritis enthaltend mindestens eine erfindungsgemäße Markersequenz und gegebenenfalls weitere Hilfs- und Zusatzstoffe.

Daher betrifft die Erfindung die Verwendung von Markersequenzen zur Stratifizierung oder Therapiesteuerung von Rheumatoider Arthritis, wobei mindestens eine Markersequenz ausgewählt aus der Gruppe der Markersequenzen SEQ ID No 1-208 und / oder der genomischen Sequenzen, die eine der Sequenzen SEQ ID No. 1-104 und / oder ein durch die Sequenzen SEQ ID No. 105-208 kodiertes Protein, Teilsequenzen oder Fragmente davon, Homologen der Sequenzen SEQ ID No. 1-208 mit mindestens 90 % Homologie zu einem zu untersuchenden Patienten bestimmt wird.

Eine weitere Ausführungsform der Erfindung betrifft die Verwendung der erfindungsgemäßen Markersequenz(en)zur Stratifizierung oder Therapiesteuerung von Rheumatoider Arthritis, dadurch gekennzeichnet, dass die Bestimmung mittels einer in-vitro Diagnose erfolgt.

Daher betrifft die Erfindung ein Verfahren zur Identifizierung von frühen RA-Patienten in medikamentöser Behandlung, die nicht auf konventionelle DMARDs, insbesondere MTX ansprechen oder nicht-ansprechen, und therapieresistent sein können.

Diese erfindungsgemäßen Marker für RA sind Gegenstand der SEQ ID No 1-3 oder 105-107 der Antigene in Tabelle 1 und Tabelle 2, die zur Stratifikation oder Therapesteuerung von RA verwendet werden können und vorraussagen bzw. diagnostizieren, ob ein RA-Patient auf eine MTX Behandlung ansprechen wird. Für die Generierung dieser Marker werden basierend auf den univariaten Ergebnissen Markerkandidat-Antigene ausgewählt, die einen p-Wert für den nichtparametrischen Mittelwertvergleich zwischen Gruppen von <0,05 aufweisen, jedoch gleichzeitig in RA Patienten reaktiv sind.

Bevorzugt sind daher erfindungsgemäß die identifizierten Marker CCDC136 (SEQ ID No. 1), HSPB1 (SEQ ID No. 2), IGFBP2 (SEQ ID No. 3) zur Feststellung einer Arzneimittel-Therapieresistenz, insbesondere einer MTX-Therapieresistenz.

Eine weitere Ausführungsform der Erfindung betrifft ein Verfahren zur Identifizierung von Markern für die Identifizierung von Patienten(sub)gruppen, die auf eine konventionelle DMARD Therapie, wie MTX, ansprechen oder nicht-ansprechen.

Marker, die mittels dieser Ausführungsform des Verfahrens gefunden werden, sind beispielsweise in Tabelle 1, in der Gruppe 5 genannt und betreffen die SEQ ID No. 4-51 und/oder SEQ ID No. 108-155.

Eine weitere Ausführungsform der Erfindung betrifft ein Verfahren zur Identifizierung von Markern für die Identifizierung von Patienten(sub)gruppen, die auf eine Kombinationstherapie von biologischen und konventionellen DMARD, wie anti-TNF Blocker und MTX, ansprechen oder nicht-ansprechen.

Marker, die mittels dieser Ausführungsform des Verfahrens gefunden werden, sind beispielsweise in Tabelle 1, in der Gruppe 6 genannt und betreffen die SEQ ID No. 52-104 und/oder SEQ ID No. 156-208.

Aufgrund der hohen klinischen und serologischen Heterogenität der RA Erkrankung ist es schwierig, mit nur einem Biomarker RA eindeutig zu diagnostizieren. Daher ist es oft erforderlich, möglichst unkorrelierte Autoantigene zu sogenannten Paneln von Markern (Biomarker Panel für RA) zu kombinieren. Beispielsweise im Rahmen der individualisierten Medizin können entsprechende Panel von Markern für RA für einzelne Patienten oder Patientengruppen individuell für den betreffenden RA Subtyp (Subgruppe) zusammengestellt werden. Deshalb ist es auch notwendig, eine Vielzahl von potentiellen Markern für RA zur Verfügung zu haben, um entsprechende Subgruppen bzw. Subtypen spezifische Marker für RA für den jeweils individuellen Fall auszuwählen. Ein entsprechendes Panel kann beispielsweise in Form einer Anordnung, eines Arrays oder auch eines oder mehrerer Beads, vorzugsweise Luminex Beads, ausgestaltet sein. Gegenstand der Erfindung ist somit eine Anordnung umfassend eine oder mehrere erfindungsgemäße Marker, ein Proteinarray umfassend eine oder mehrere erfindungsgemäße Marker, ein Bead (Kügelchen oder Plättchen) umfassend eine oder mehrere erfindungsgemäße Marker.

Die erfindungsgemäßen Markersequenzen konnten mittels differentiellem Screening von Proben von gesunden Probanden mit Patientenproben mit Rheumatoider Arthritis identifiziert werden. Die erfindungsgemäßen Markersequenzen wurden exprimiert und nach Kopplung der exprimierten Markersequenz-Kandidaten an Luminex-Beads mit Hilfe der Luminex-Beads validiert, z.T. vergleichend gegen bekannte Biomarker für Rheumatoide Arthritis. Dadurch konnten hochspezifische Markersequenzen für Rheumatoide Arthritis identifiziert werden.

"Beads" (Perlen, Kügelchen, ursprünglich auch Latex-Partikel genannt) bezeichnen sogenannte Mikrosphären oder Mikropartikel, die als Träger für Biomoleküle in Tests und Assays eingesetzt werden. Für Tests und Assays werden uniforme (etwa gleichgroße) Mikropartikel benötigt, die über spezielle chemische Verfahren hergestellt werden. Diese Verfahren sind dem Fachmann bekannt. Beads für unterschiedliche Anwendungen sind auch kommerziell erhältlich (z.B. Fa. Progen Biotechnik GmbH). Beads können aus unterschiedlichen Materialien bestehen, beispielsweise Glas, Polystyrol, PMMA und verschiedenen anderen, zum Teil auch Kopolymeren. Beads können mit unterschiedlichen Farbstoffen oder Farbstoffgemischen markiert werden und mit Coatings versehen werden. An ihre Oberfläche können Biomoleküle gekoppelt werden. Dafür stehen unterschiedliche Kopplungsmethoden zur Verfügung, die dem Fachmann bekannt sind, beispielsweise Adsorption oder kovalente Kopplung. Die Oberfläche der Beads kann modifiziert sein, so dass eine gerichtete Kopplung der Biomoleküle auf der Bead-Oberfläche, z.B. in Verbindung mit Spacern, Tags oder speziellen Modifikationen möglich ist und wodurch die analytische Sensitivität weiter erhöht werden kann.

Der Begriff "Rheumatoide Arthritis (RA)" ist z.B. nach Pschyrembel, de Gruyter, 261. Auflage (2007), Berlin definiert. Erfindungsgemäß umfasst ist ebenfalls die "juvenile idiopathische Arthritis " (ICD-10: M08.-. Abk.: JIA. Ältere Synonyme: Juvenile rheumatoide Arthritis, Juvenile chronische Arthritis, Morbus Still oder volkstümlicher: kindliches Rheuma), die eine Sammelbezeichnung für eine Reihe von vorwiegend gelenkbefallenden Erkrankungen (Arthritis) des rheumatischen Formenkreises im Kindesalter (juvenil) (Definition z.B. nach Pschyrembel, de Gruyter, 261. Auflage (2007), Berlin).

In einer bevorzugten Ausführungsform erfolgt die Bestimmung der Markersequenzen außerhalb des menschlichen Körpers und die Bestimmung erfolgt in einer ex vivo / in vitro Diagnose.

"Diagnose" im Sinne dieser Erfindung bedeutet die positive Feststellung von Rheumatoider Arthritis mittels der erfindungsgemäßen Markersequenzen sowie die Zuordnung der Patienten zur Indikation Rheumatoide Arthritis. Der Begriff der Diagnose umfasst die medizinische Diagnostik und diesbezügliche Untersuchungen, insbesondere die in-vitro Diagnostik und Labordiagnostik, ebenfalls Proteomics und Nukleinsäureblots. Weitere Untersuchungen können zur Absicherung und zum Ausschluss anderen Krankheiten vonnöten sein. Daher umfasst der Begriff Diagnose ebenfalls die Differentialdiagnose von Rheumatoider Arthritis mittels der erfindungsgemäßen Markersequenzen sowie die Prognose bei festgestellter Rheumatoider Arthritis.

Ferner betrifft die Erfindung ein Verfahren zum Stratifizieren, insbesondere zur Risikostratifizierung und / oder Therapiesteuerung eines Patienten mit Rheumatoider Arthritis, beispielsweise bei einem Patienten mit einem sehr frühen Stadium von RA, dass nicht mittels des Markers CCP nachgewiesen werden kann, wobei mindestens eine erfindungsgemäße Markersequenz an einem zu untersuchenden Patienten bestimmt wird. Umfasst ist ebenfalls die Stratifizierung der Patienten mit Rheumatoider Arthritis in neue oder etablierte Subgruppen innerhalb der Erkrankung Rheumatoide Arthritis, sowie die sinnvolle Auswahl von Patientengruppen für die Auswahl einer geeigneten Therapie mit bestimmten Wirkstoffen. Der Begriff Therapiesteuerung umfasst erfindungsgemäß die Einteilung von Patienten in Responder und Nicht-Responder bezüglich einer medikamentösen Therapie oder dessen Therapieverlauf.

Erfindungsgemäß bedeutet "Stratifizieren oder Therapiesteuerung", insbesondere Vorhersage/Prognose und Überwachung des Ansprechen (Response) oder Nicht-Ansprechen (Non-Response) auf ein Medikament/Arzneimittel zur Behandlung der RA oder eine geänderte Therapie als auch die Nachsorge.

"Stratifizieren oder Therapiesteuerung" im Sinne dieser Erfindung bedeutet, dass das erfindungsgemäße Verfahren Entscheidungen zur Behandlung und Therapie des Patienten erlaubt, sei es Hospitalisierung des Patienten, Einsatz, Wirkung und / oder Dosierung eines oder mehrerer Arzneimittel, eine therapeutische Maßnahme oder die Überwachung eines Krankheitsverlaufes sowie des Therapieverlaufs bzw. Ätiologie oder Klassifizierung von RA, z.B. in einen neuen oder bestehenden Subtyp oder die Differenzierung von RA und den betreffenden Patienten.

In einer weiteren Ausführungsform der Erfindung umfasst der Begriff "Stratifizierung" insbesondere die Risikostratifizierung mit der Prognose eines "outcome" eines nachteiligen gesundheitlichen Ereignisses.

Im Rahmen dieser Erfindung wird unter "Patient" ein beliebiger Proband - Mensch oder Säugetier - verstanden, mit der Maßgabe, dass der Proband auf Rheumatoide Arthritis untersucht wird, insbesondere ein Therapieerfolg oder Therapieverbesserung oder mit der Maßgabe, dass der Proband bzw. das Individuum auf RA untersucht wird, und Medikamente zur Behandlung von RA erhält bzw. verabreicht bekommt, wie beispielsweise mittels einer Basistherapie (supra).

Der Begriff "Markersequenzen" im Sinne dieser Erfindung bedeutet, dass die Nukeinsäuresequenz, beispielsweise die mRNA, cDNA oder das jeweils daraus erhältliche Polypeptid oder Protein signifikant für Rheumatoide Arthritis sind. Beispielsweise können die mRNA oder cDNA oder das jeweils daraus erhältliche Polypeptid oder Protein eine Wechselwirkung mit Substanzen aus der Körperflüssigkeit oder Gewebeauszug eines Patienten mit Rheumatoider Arthritis aufweisen (z.B. (Auto)Antigen (Epitop) / (Auto)Antikörper (Paratop) Wechselwirkung). In einer besonders bevorzugten Ausführungsform der Erfindung ist der Marker für RA ein Antigen oder ein Teil eines Antigens oder kodiert für ein Antigen oder für einen Teil eines Antigens.

Im Sinne der Erfindung bedeutet "wobei mindestens eine Markersequenz ausgewählt aus der Gruppe der Markersequenzen SEQ ID No. 1-208 und / oder der genomischen Sequenzen, die eine der Sequenzen SEQ ID No. 1-104 und / oder ein durch die Sequenzen SEQ ID No. 105-208 kodiertes Protein, Teilsequenzen oder Fragmente davon, Homologen der Sequenzen SEQ ID No. 1-208 mit mindestens 90 % Homologie zu einem zu untersuchenden Patienten bestimmt wird", dass eine Wechselwirkung zwischen der Körperflüssigkeit oder dem Gewebeauszug eines Patienten und der/den erfindungsgemäßen Markersequenzen nachgewiesen wird. Eine solche Wechselwirkung ist z.B. eine Bindung, insbesondere eine bindende Substanz an mindestens eine der erfindungsgemäßen Markersequenzen oder im Fall einer cDNA die Hybridisierung mit einer geeigneten Substanz unter gewählten Bedingungen, insbesondere stringenten Bedingungen (z.B. wie üblich definiert in J. Sambrook, E.F. Fritsch, T. Maniatis (1989), Molecular cloning: A laboratory manual, 2nd Edition, Cold Spring Habor Laboratory Press, Cold Spring Habor, USA oder Ausubel, "Current Protocols in Molecular Biology", Green Publishing Associates and Wiley Interscience, N.Y. (1989)). Ein Beispiel für stringente Hybridisierungsbedingungen ist: Hybridisierung in 4 x SSC bei 65° C (alternativ in 50% Formamid und 4 X SSC bei 42° C), gefolgt von mehreren Waschschritten in 0,1 x SSC bei 65°C für insgesamt etwa eine Stunde. Ein Beispiel für wenig stringente Hybridisierungsbedingungen ist Hybridisierung in 4 x SSC bei 37° C, gefolgt von mehreren Waschritten in 1 x SSC bei Raumtemperatur.

Solche Substanzen sind erfindungsgemäß Bestandteil einer Körperflüssigkeit, insbesondere Blut, Vollblut, Blutplasma, Blutserum, Patientenserum, Urin, Cerebrospinalflüssigkeit, Synovialflüssigkeit oder eines Gewebeauszuges des Patienten.

Die Substanzen aus der Körperflüssigkeit oder Gewebeauszug treten entweder nur oder zumindest verstärkt bei RA auf beziehungsweise werden exprimiert, wohingegen diese Substanzen bei Patienten ohne RA oder Gesunden nicht oder zumindest in geringerem Maße (geringere Menge, geringerer Konzentration) vorhanden sind. Marker für RA können sich andererseits auch dadurch auszeichnen, dass sie eine Wechselwirkung mit Substanzen aus der Körperflüssigkeit oder Gewebeauszug von Patienten mit RA eingehen, weil diese Substanzen nicht mehr oder zumindest in deutlich geringerer Menge/Konzentration bei RA auftreten beziehungsweise exprimiert werden, wohingegen diese Substanzen bei Patienten ohne RA vorhanden oder zumindest in deutlich höherem Maße vorhanden sind. Marker für RA können auch in gesunden Probanden vorhanden sein, jedoch verändert sich ihre Menge (Konzentration) beispielsweise bei der Entstehung, Etablierung und Therapie von RA. Ein oder mehrere Marker können auf diese Weise ein Profil von Substanzen aus Körperflüssigkeit und Gewebeauszug abbilden, beispielsweise ein RA assoziiertes Autoantikörperprofil des betreffenden Patienten. Erfindungsgemäße Marker sind Biomarker für RA.

Autoantikörperprofile umfassen die Menge an einem oder mehreren Autoantikörpern deren Vorkommen/Expression mit der Entstehung und/oder Etablierung von RA einhergehen. Autoantikörperprofile umfassen somit einerseits die Zusammensetzung, d.h. es werden beispielsweise ein oder mehrere Autoantikörper nur bei RA exprimiert, und anderseits die Menge/Konzentration einzelner Autoantikörper, d.h. die Menge/Konzentration einzelner Autoantikörper verändert sich bei der Entstehung und Etablierung von RA. Diese Veränderungen können mit Hilfe der erfindungsgemäßen Markersequenzen nachgewiesen werden.

Die erfindungsgemäßen Markersequenzen SEQ ID No. 1-208 sind Gegenstand der Tabelle 1 und können durch den jeweilig zitierten Datenbankeintrag (auch mittels Internet: http://www.ncbi.nlm.nih.gov/) eindeutig identifiziert werden (siehe RefSeq Accession bzw. GI Accession).

Daher betrifft die Erfindung ebenfalls die Volllängesequenzen der erfindungsgemäßen Markersequenzen und die Markersequenzen wie in den Tabellen über die bekannten Datenbankeinträge definiert sowie die im anliegenden Sequenzprotokoll angegebenen Markersequenzen.

Weiterhin umfasst sind ebenfalls analoge Ausführungsformen der Markersequenzen, insbesondere der Nukleinsäuresequenzen SEQ ID No. 1-104 und der Proteinsequenzen SEQ ID No. 105-208.

In einer weiteren Ausführungsform der Erfindung sind Markersequenzen bevorzugt, die P-Werte kleiner oder gleich 0.006 aufweisen, vorzugsweise kleiner oder gleich 0.001 oder kleiner oder gleich 0.0001, besonders bevorzugt kleiner oder gleich 0.00001 (siehe Tabellen).

In einer weiteren Ausführungsform der Erfindung sind Homologe der erfindungsgemäßen Markersequenzen umfasst. Insbesondere solche Homologen, die eine Identität von 70 %, 80 % oder 85 %, vorzugsweise 90 %, 91 %, 92 %, 93 %, 94 % oder 95% Identität, insbesondere 96 %, 97 %, 98 %, 99 % oder mehr Identität mit den erfindungsgemäßen Markersequenzen aufweisen und für die erfindungsgemäße Verwendung - den Nachweis von Rheumatoider Arthritis geeignet sind (sog. "Homologe", homologe Markersequenzen). Homologe können Protein- oder Nukleinsäuresequenzen sein.

Teilsequenzen oder Fragmente sind solche Sequenzen, die 50 bis 100 Nukleotide oder Aminosäuren, vorzugsweise 70-120 Nukleotide oder Aminosäuren, besonders bevorzugt 100 bis 200 Nukleotide oder Aminosäuren einer der Markersequenzen SEQ ID No. 1-208 aufweisen.

Erfindungsgemäß umfassen die Markersequenzen auch solche Modifikationen der Nukleotidsequenz, beispielsweise der cDNA-Sequenz und der entsprechenden Aminosäuresequenz, wie chemische Modifikation, beispielsweise Citrullinierung, Acetylierung, Phosphorylierung, Glykosilierung oder polyA-Strang und weiteren dem Fachmann einschlägig bekannte Modifikationen.

In einer weiteren Ausführungsform kann die jeweilige Markersequenz in unterschiedlichen Mengen in einem oder mehreren Bereichen auf einem festen Träger, beispielsweise einem Bead repräsentiert sein. Dies erlaubt eine Variation der Sensitivität. Die Bereiche können jeweils eine Gesamtheit von Markersequenzen aufweisen, d.h. eine genügende Zahl an verschiedenen Markersequenzen, insbesondere 2 bis 5 oder 10 oder mehr Markersequenzen und ggfs. weitere Nukleinsäuren und/oder Proteine, insbesondere Biomarker. Bevorzugt sind jedoch mindestens 96 bis 25.000 (numerisch) oder mehr aus verschiedenen oder gleichen Markersequenzen und weiteren Nukleinsäuren und/oder Proteinen, insbesondere Biomarkern. Weiterhin bevorzugt sind mehr als 2.500, besonders bevorzugt 10.000 oder mehr verschiedene oder gleiche Markersequenzen und ggfs. weitere Nukleinsäuren und/oder Proteine, insbesondere Biomarker.

Im Rahmen dieser Erfindung bedeutet "Anordnung" oder "Panel" synonym "Array" und sofern dieser "Array" zur Identifizierung von zu bindenden Substanzen an Markersequenzen verwendet wird, ist hierunter ein "Assay" oder eine diagnostische Vorrichtung zu verstehen. In einer bevorzugten Ausführungsform ist die Anordnung derart gestaltet, dass die auf der Anordnung repräsentierten Markersequenzen in Form eines Gitters auf einem festen Träger vorliegen. Ferner sind solche Anordnungen bevorzugt, die eine hochdichte (high-density) Anordnung von Markersequenzen erlauben und die Markersequenzen gespottet werden. Solche hochdichte gespotteten Anordnungen sind beispielsweise in der WO 99/57311 und WO 99/57312 offenbart und können vorteilhaft in einem robotergestützten automatisierten High-Throughput Verfahren zur Anwendung kommen.

Im Rahmen dieser Erfindung umfasst der Begriff "Assay" oder diagnostische Vorrichtung ebenfalls solche Ausführungsformen einer Vorrichtung, wie ELISA (z.B. einzelne Wells einer Mikrotiterplatte werden mit den erfindungsgemäßen Markersequenzen oder Kombinationen von Markersequenzen beschichtet, ggfs. robotergestützt in die einzelnen Wells der Mikrotiterplatte aufgebracht; Beispiele sind diagnostische ELISA-Kits der Fa. Phadia oder Multiplex-ELISA-Kits "Searchlight" der Fa. Pierce/Thermo Fisher Scientific), Beadbased Assay (spektral unterscheidbare bead-Populationen werden mit Markersequenzen/Kombinationen von Markersequenzen beschichtet. Die Patientenprobe wird mit dieser bead-Population inkubiert und gebundene (Auto-)-Antikörper werden mittels eines weiteren Fluoreszenz-markierten Sekundärantikörpers oder ein Detektionsreagenz über Messung der Fluoreszenz nachgewiesen; z. B. Borrelia IgG-Kit oder Athena-Multilyte der Fa. Multimetrix), Line Assay (erfindungsgemäßen Markersequenzen oder Kombinationen von Markersequenzen werden robotergestützt auf Membranen immobilisiert, welche mit der Patientenprobe untersucht beziehungsweise inkubiert werden; Beispiel "Euroline" der Fa. Euroimmun AG), Western Blot (Beispiel "Euroline-WB" der Fa. Euroimmun AG), immunchromatographische Verfahren (z.B. Lateral Flow Immunoassays; Markersequenzen bzw. Kombinationen von Markersequenzen werden auf Teststreifen (Membranen, US 5,714,389 u.v.a) immobilisiert; Beispiel "One Step HBsAg" Test Device von Acon Laboratories) oder ähnliche immunologische Single- oder Multiplex-Nachweisverfahren.

Daher betrifft die Erfindung ebenfalls die Aufgabe eine diagnostische Vorrichtung oder einen Assay, insbesondere einen Proteinbiochip, bereitzustellen, der für die Rheumatoide Arthritis eine Stratifizierung und Therapiesteuerung erlaubt.

Zur Lösung dieser Aufgabe sind die erfindungsgemäßen Markersequenzen der Anordnung oder Panel auf einen festen Träger fixiert, vorzugsweise jedoch gespottet oder immobilisiert oder aufgedruckt, d.h. reproduzierbar aufgebracht. Ein oder mehrere Markersequenzen können mehrfach in der Gesamtheit aller Markersequenzen präsent sein und in unterschiedlichen Mengen bezogen auf einen Spot vorliegen. Ferner können die Markersequenzen auf dem festen Träger standardisiert sein (z.B. mittels serieller Verdünnungsreihen von z.B. Humanglobulinen als interne Kalibratoren zur Datennormalisierung und quantitativen Auswertung).

Daher betrifft die Erfindung einen Assay oder Proteinbiochip oder ein oder mehrere Beads (Bead-basierter Assay) bestehend aus einer Anordnung oder Panel enthaltend erfindungsgemäße Markersequenzen.

In einer weiteren Ausführungsform liegen die Markersequenzen als Klone vor. Solche Klone können beispielsweise mittels einer erfindungsgemäßen cDNA-Expressionsbibliothek erhalten werden (Büssow et al. 1998 (supra)). In einer bevorzugten Ausführungsform werden solche Expressionsbibliotheken enthaltend Klone mittels Expressionsvektoren aus einer exprimierenden cDNA Bibliothek bestehend aus den cDNA Markersequenzen erhalten. Diese Expressionsvektoren enthalten vorzugsweise induzierbare Promotoren. Die Induktion der Expression kann z.B. mittels eines Induktors, solche wie IPTG, erfolgen. Geeignete Expressionsvektoren sind beschrieben in Terpe et al. (Terpe T Appl Microbiol Biotechnol. 2003 Jan; 60(5):523-33).

Expressionsbibliotheken sind dem Fachmann bekannt, diese können nach Standardwerken, wie Sambrook et al, "Molecular Cloning, A laboratory handbook, 2nd edition (1989), CSH press, Cold Spring Harbor, New York hergestellt werden. Weiterhin bevorzugt sind solche Expressionsbibliotheken, die gewebespezifisch sind (z.B. humanes Gewebe, insbesondere humane Organe). Ferner sind erfindungsgemäß ebenfalls solche Expressionsbibliotheken umfasst, die mittels exon-trapping erhalten werden können. Statt Expressionsbibliothek kann synonym von einer Expressionsbank gesprochen werden.

Weiterhin bevorzugt sind Proteinbiochips oder Beads oder entsprechende Expressionsbibliotheken, die keine Redundanz aufweisen (so genannte: Uniclone®-Bibliothek) und nach den Lehren der WO 99/57311 und WO 99/57312 beispielsweise hergestellt werden können. Diese bevorzugten Uniclone-Bibliotheken weisen einen hohen Anteil an nicht-fehlerhaften vollständig exprimierten Proteinen einer cDNA-Expressionsbibliothek auf.

Im Rahmen dieser Erfindung können die Klone ebenfalls nicht abschließend solche sein, wie transformierte Bakterien, rekombinante Phagen oder transformierte Zellen von Säugern, Insekten, Pilzen, Hefen oder Pflanzen.

Die Klone werden auf einen festen Träger fixiert, gespottet oder immobilisiert.

Daher betrifft die Erfindung eine Anordnung, wobei die Markersequenzen als Klone vorliegen.

Zusätzlich können die Markersequenzen in der jeweiligen Form in Form eines Fusionsproteins vorliegen, welches beispielsweise mindestens ein Affinitätsepitop oder "Tag" enthält. Der Tag kann ein solcher sein wie wie c-myc, His-Tag, Arg-tag, FLAG, alkalische Phosphatase, V5-Tag, T7-Tag oder Strep-Tag, HAT-tag, NusA, S-tag, SBP-tag, Thioredoxin, DsbA, ein Fusionsprotein, vorzugsweise eine Cellulose-bindende Domäne, grünfluoreszierendes Protein, Maltose bindendes Protein, calmodulin-bindendes Protein, Glutathione S-transferase oder lacZ enthalten.

Eine Markersequenz kann sich auch aus mehreren einzelnen Markersequenzen zusammensetzen. Dies kann die Klonierung einzelner Fragmente zu einem großen gemeinsamen Fragment und die Expression dieses kombinierten Fragmentes beinhalten.

In sämtlichen Ausführungsformen umfasst der Begriff "fester Träger" Ausführungen wie einen Filter, eine Membran, ein magnetisches oder Fluorophor-markiertes Kügelchen, ein Silizium-Wafer, Glas, Metall, Kunststoff, ein Chip, ein massenspektrometrisches Target oder eine Matrix. Ein Filter und Beads sind jedoch erfindungsgemäß bevorzugt.

Als Filter ist weiterhin PVDF, Nitrocellulose oder Nylon bevorzugt (z.B. Immobilon P Millipore, Protran Whatman, Hybond N+ Amersham).

In einer weiteren bevorzugten Ausführungsform der erfindungsgemäßen Anordnung entspricht diese einem Gitter, dass die Größenordnung einer Mikrotiterplatte (8-12 Wells Streifen, 96 Wells, 384 Wells oder mehr), eines Silizium-Wafers, eines Chips, eines massenspektrometrischen Targets oder einer Matrix besitzt.

In einer weiteren bevorzugten Ausführungsform werden Kügelchen sogenannte Beads als Träger verwendet. Vorzugsweise werden dabei Bead-basierte multiplex Assays verwendet. Die Analyse und Auswertung der Bead-basierten Assays kann beispielsweise mit einem Luminex-Analysesystem erfolgen, welches auf der Methode der Durchflusszytometrie unter Verwendung zweier unterschiedlicher Laser durchgeführt wird.

Während die Messungen auf planaren Proteinarrays lediglich einen dynamischen Bereich von 1,5 - 2 Größenordnungen (10er Potenzen) bieten, kann durch die Verwendung von Luminex-Beads ein dynamischer Bereich von 3,5-4 Größenordnungen abgedeckt werden. Wobei auch die Messungen in den niedrigen Responsbereichen sehr gute Variationskoeffizienten (VKs), d.h. nicht mehr als 10 % liefern.

Während die Messungen auf planaren Proteinarrays lediglich Variationskoeffizienten (VKs) von 10 bis 25 % (intra-Array Vergleich) bzw. 10 bis 50 % (inter-Array Vergleich) liefern, befinden sich die VKs der Luminex-Messungen zwischen 3 bis 10 %. Dadurch ergibt sich eine Assayqualität, die kommerzielle ELISAs im Allgemeinen nicht erreichen. Die bekannten Nachteile (limitierte Plexing-Rate durch Interferenz unterschiedlicher Detektionsantikörper) für Luminex-basierte Analyse- und Diagnostikverfahren treffen auf das UNIarray-Konzept nicht zu, da als Detektionssonde lediglich ein einziges fluoreszenzmarkiertes anti-human IgG aus Ziege, Schaf oder Maus eingesetzt wird. Durch den Transfer des UNIarray-Konzeptes auf Luminex (also Bead-basierte Protein-Arrays) können zusätzlich mehrere Geräte eingespart, d.h. Protein-Drucker, Hybridisiermaschine und Array-Reader, und durch ein Gerät ersetzt werden. Dabei ist das UNIarray-Konzept nicht an Luminex gebunden, sondern kann auch auf anderen Plattformen wie z.B. Randox, VBC-Genomics etc eingesetzt werden. Die hohe Messgenauigkeit und die niedrigen VKs der Einzelmessungen erlauben den Einsatz besserer und neuer statistischer Verfahren zur Identifizierung von potenten Einzelmarkern sowie zur schnellen Aussortierung von Falschpositiven.

Die Visualisierung erfindungsgemäßer Protein-Protein-Wechselwirkungen (z.B. Protein an Markersequenz, wie Antigen/Antikörper) oder entsprechende "Mittel zum Nachweis des Bindungserfolges" kann beispielsweise mittels Fluoresenzmarkierung, Biotiniylierung, Radio-Isotopen-Markierung oder kolloidale Gold- oder Latex-Partikel-Markierung in üblicher Weise erfolgen. Ein Nachweis von gebundenen Antikörpern erfolgt mit Hilfe von sekundären Antikörpern, die mit handelsüblichen Reportermolekülen markiert sind (z.B. Cy-, Alexa-, Dyomics, FITC- oder ähnliche Fluoreszenzfarbstoffe, kolloidale Gold- oder Latex-Partikel), oder mit Reporter-Enzymen wie alkalischer Phosphatase, Meerrettichperoxidase, usw. und den entsprechenden colorimetrischen, fluoreszenten oder chemolumineszenten Substraten. Eine Auslesung erfolgt z.B. mittels eines Microarray-Laserscanners, einer CCD-Kamera oder visuell.

### Beispiele:

**Tabelle 1: Markersequenzen (Antigene)**

| **Nr** | **Gene ID** | **Gene Symbol** | **Gene Name** | **Gruppe** |
|---|---|---|---|---|
| **1** | 64753 | CCDC136 | coiled-coil domain containing 136 | Gruppe 1 |
| **2** | 3281 | HSBP1 | heat shock factor binding protein 1 | Gruppe 1 |
| **3** | 3485 | IGFBP2 | insulin-like growth factor binding protein 2, 36kDa | Gruppe 1 |
| **4** | 10476 | ATP5H | ATP synthase, H+ transporting, mitochondrial Fo complex, subunit d | Gruppe 5 |
| **5** | 7812 | CSDE1 | cold shock domain containing E1, RNAbinding | Gruppe 5 |
| **6** | 1485 | CTAG1B | cancer/testis antigen 1B | Gruppe 5 |
| **7** | 8320 | EOMES | eomesodermin | Gruppe 5 |
| **8** | 10961 | ERP29 | endoplasmic reticulum protein 29 | Gruppe 5 |
| **9** | 84893 | FBXO18 | F-box protein, helicase, 18 | Gruppe 5 |
| **10** | 3059 | HCLS1 | hematopoietic cell-specific Lyn substrate 1 | Gruppe 5 |
| **11** | 3182 | HNRNPAB | heterogeneous nuclear ribonucleoprotein A/B | Gruppe 5 |
| **12** | 3798 | KIF5A | kinesin family member 5A kinesin | Gruppe 5 |
| **13** | 8079 | MLF2 | myeloid leukemia factor 2 | Gruppe 5 |
| **14** | 60560 | NAA35 | N(alpha)-acetyltransferase 35, NatC auxiliary subunit | Gruppe 5 |
| **15** | 8473 | OGT | O-linked N-acetylglucosamine (GlcNAc) transferase | Gruppe 5 |
| **16** | 5174 | PDZK1 | PDZ domain containing 1 | Gruppe 5 |
| **17** | 23645 | PPP1R15A | protein phosphatase 1, regulatory subunit 15A | Gruppe 5 |
| **18** | 64062 | RBM26 | RNA binding motif protein 26 | Gruppe 5 |
| **19** | 23170 | TTLL12 | tubulin tyrosine ligase-like family member 12 | Gruppe 5 |
| **20** | 91544 | UBXN11 | UBX domain protein 11 | Gruppe 5 |
| **21** | 6944 | VPS72 | vacuolar protein sorting 72 homolog (S. cerevisiae) | Gruppe 5 |
| **22** | 16303 3 | ZNF579 | zinc finger protein 579 | Gruppe 5 |
| **23** | 23625 | FAM89B | family with sequence similarity 89, member B | Gruppe 5 |
| **24** | 27344 | PCSK1N | proprotein convertase subtilisin/kexin type 1 inhibitor | Gruppe 5 |
| **25** | 64762 | GAREM | GRB2 associated, regulator of MAPK1 | Gruppe 5 |
| **26** | 30 | ACAA1 | acetyl-CoA acyltransferase 1 | Gruppe 5 |
| **27** | 11093 | ADAMTS13 | ADAM metallopeptidase with thrombospondin type 1 motif, 13 | Gruppe 5 |
| **28** | 204 | AK2 | adenylate kinase 2 | Gruppe 5 |
| **29** | 211 | ALAS1 | 5'-aminolevulinate synthase 1 | Gruppe 5 |
| **30** | 90416 | C15orf57 | chromosome 15 open reading frame 57 | Gruppe 5 |
| **31** | 51531 | TRMO | tRNA methyltransferase O | Gruppe 5 |
| **32** | 1108 | CHD4 | chromodomain helicase DNA binding protein 4 | Gruppe 5 |
| **33** | 22982 | DIP2C | disco-interacting protein 2 homolog C | Gruppe 5 |
| **34** | 9454 | HOMER3 | homer scaffolding protein 3 | Gruppe 5 |
| **35** | 3490 | IGFBP7 | insulin-like growth factor binding protein 7 | Gruppe 5 |
| **36** | 10445 | MCRS1 | microspherule protein 1 | Gruppe 5 |
| **37** | 11295 0 | MED8 | mediator complex subunit 8 | Gruppe 5 |
| **38** | 65003 | MRPL11 | mitochondrial ribosomal protein L11 | Gruppe 5 |
| **39** | 7469 | NELFA | negative elongation factor complex member A | Gruppe 5 |
| **40** | 84081 | NSRP1 | nuclear speckle splicing regulatory protein 1 | Gruppe 5 |
| **41** | 81572 | PDRG1 | p53 and DNA-damage regulated 1 | Gruppe 5 |
| **42** | 5494 | PPM1A | protein phosphatase, Mg2+/Mn2+ dependent, 1A | Gruppe 5 |
| **43** | 5536 | PPP5C | protein phosphatase 5, catalytic subunit | Gruppe 5 |
| **44** | 9727 | RAB11FIP3 | RAB11 family interacting protein 3 (class II) | Gruppe 5 |
| **45** | 10111 | RAD50 | RAD50 homolog, double strand break repair protein | Gruppe 5 |
| **46** | 6449 | SGTA | small glutamine-rich tetratricopeptide repeat (TPR)-containing, alpha | Gruppe 5 |
| **47** | 11684 1 | SNAP47 | synaptosomal-associated protein, 47kDa | Gruppe 5 |
| **48** | 10290 | SPEG | SPEG complex locus | Gruppe 5 |
| **49** | 10422 | UBAC1 | UBA domain containing 1 | Gruppe 5 |
| **50** | 65109 | UPF3B | UPF3 regulator of nonsense transcripts homolog B (yeast) | Gruppe 5 |
| **51** | 8882 | ZPR1 | ZPR1 zinc finger | Gruppe 5 |
| **52** | 39 | ACAT2 | acetyl-CoA acetyltransferase 2 | Gruppe 6 |
| **53** | 348 | APOE | apolipoprotein E | Gruppe 6 |
| **54** | 12806 1 | C1orf131 | chromosome 1 open reading frame 131 | Gruppe 6 |
| **55** | 79714 | CCDC51 | coiled-coil domain containing 51 | Gruppe 6 |
| **56** | 6249 | CLIP1 | CAP-GLY domain containing linker protein 1 | Gruppe 6 |
| **57** | 30827 | CXXC1 | CXXC finger protein 1 | Gruppe 6 |
| **58** | 9704 | DHX34 | DEAH (Asp-Glu-Ala-His) box polypeptide 34 | Gruppe 6 |
| **59** | 57572 | DOCK6 | dedicator of cytokinesis 6 | Gruppe 6 |
| **60** | 84444 | DOT1L | DOT1-like histone H3K79 methyltransferase | Gruppe 6 |
| **61** | 23301 | EHBP1 | EH domain binding protein 1 | Gruppe 6 |
| **62** | 8661 | EIF3A | eukaryotic translation initiation factor 3, subunit A | Gruppe 6 |
| **63** | 1982 | EIF4G2 | eukaryotic translation initiation factor 4 gamma, 2 | Gruppe 6 |
| **64** | 63877 | FAM204A | family with sequence similarity 204, member A | Gruppe 6 |
| **65** | 54865 | GPATCH4 | G patch domain containing 4 | Gruppe 6 |
| **66** | 2922 | GRP | gastrin-releasing peptide | Gruppe 6 |
| **67** | 84064 | HDHD2 | haloacid dehalogenase-like hydrolase domain containing 2 | Gruppe 6 |
| **68** | 80895 | ILKAP | integrin-linked kinase-associated serine/threonine phosphatase | Gruppe 6 |
| **69** | 23479 | ISCU | iron-sulfur cluster assembly enzyme | Gruppe 6 |
| **70** | 57498 | KIDINS220 | kinase D-interacting substrate, 220kDa | Gruppe 6 |
| **71** | 3875 | KRT18 | keratin 18, type I | Gruppe 6 |
| **72** | 29094 | LGALSL | lectin, galactoside-binding-like | Gruppe 6 |
| **73** | 79888 | LPCAT1 | lysophosphatidylcholine acyltransferase 1 | Gruppe 6 |
| **74** | 4357 | MPST | mercaptopyruvate sulfurtransferase | Gruppe 6 |
| **75** | 10528 | NOP56 | NOP56 ribonucleoprotein | Gruppe 6 |
| **76** | 51602 | NOP58 | NOP58 ribonucleoprotein | Gruppe 6 |
| **77** | 9315 | NREP | neuronal regeneration related protein | Gruppe 6 |
| **78** | 23762 | OSBP2 | oxysterol binding protein 2 | Gruppe 6 |
| **79** | 55593 | OTUD5 | OTU deubiquitinase 5 | Gruppe 6 |
| **80** | 79668 | PARP8 | poly (ADP-ribose) polymerase family, member 8 | Gruppe 6 |
| **81** | 23089 | PEG10 | paternally expressed 10 | Gruppe 6 |
| **82** | 9360 | PPIG | peptidylprolyl isomerase G (cyclophilin G) | Gruppe 6 |
| **83** | 84687 | PPP1R9B | protein phosphatase 1, regulatory subunit 9B | Gruppe 6 |
| **84** | 5589 | PRKCSH | protein kinase C substrate 80K-H | Gruppe 6 |
| **85** | 5834 | PYGB | phosphorylase, glycogen; brain | Gruppe 6 |
| **86** | 11758 4 | RFFL | ring finger and FYVE-like domain containing E3 ubiquitin protein ligase | Gruppe 6 |
| **87** | 9025 | RNF8 | ring finger protein 8, E3 ubiquitin protein ligase | Gruppe 6 |
| **88** | 6091 | ROBO1 | roundabout guidance receptor 1 | Gruppe 6 |
| **89** | 6240 | RRM1 | ribonucleotide reductase M1 | Gruppe 6 |
| **90** | 22937 | SCAP | SREBF chaperone | Gruppe 6 |
| **91** | 23256 | SCFD1 | sec1 family domain containing 1 | Gruppe 6 |
| **92** | 6382 | SDC1 | syndecan 1 | Gruppe 6 |
| **93** | 10483 | SEC23B | Sec23 homolog B, COPII coat complex component | Gruppe 6 |
| **94** | 6576 | SLC25A1 | solute carrier family 25 (mitochondrial carrier; citrate transporter), member 1 | Gruppe 6 |
| **95** | 23384 | SPECC1L | sperm antigen with calponin homology and coiled-coil domains 1-like | Gruppe 6 |
| **96** | 23635 | SSBP2 | single-stranded DNA binding protein 2 | Gruppe 6 |
| **97** | 6612 | SUMO3 | small ubiquitin-like modifier 3 | Gruppe 6 |
| **98** | 84260 | TCHP | trichoplein, keratin filament binding | Gruppe 6 |
| **99** | 9804 | TOMM20 | translocase of outer mitochondrial membrane 20 homolog (yeast) | Gruppe 6 |
| **100** | 55850 | USE1 | unconventional SNARE in the ER 1 homolog (S. cerevisiae) | Gruppe 6 |
| **101** | 89891 | WDR34 | WD repeat domain 34 | Gruppe 6 |
| **102** | 54521 | WDR44 | WD repeat domain 44 | Gruppe 6 |
| **103** | 23036 | ZNF292 | zinc finger protein 292 | Gruppe 6 |
| **104** | 6234 | RPS28 | ribosomal protein S28 | Gruppe 6 |

**Tabelle 2: 3 Marker zur Frühdiagnose von RA und Identifizierung von MTX Therapieversagern**

| | | | | **Frühe RA (HitHard)** | | |
|---|---|---|---|---|---|---|
| **Nr** | **Gruppe** | **gene ID** | **Gene Symbol** | **p-value** | **Foldchange** | **Sens. (%) bei 90% Spez.** |
| 1 | Gruppe 1 | 64753 | CCDC136 | 0,031 | 1,7 | 14 |
| 2 | Gruppe 1 | 3281 | HSBP1 | 0,002 | 1,3 | 25 |
| 3 | Gruppe 1 | 3485 | IGFBP2 | 0,0004 | 1,5 | 19 |

Tabelle 2 enthält 3 Antigene, die besonders dazu geeignet sind, RA Patienten zu identifizieren, die ACPA negativ sind und wahrscheinlich nicht von einer MTX Basistherapie profitieren werden.

**Tabelle 3: 51 Marker zur Steuerung von MTX-Therapie**

| NR: Marker erhöht bei MTX Therapieresistenz (Therapie-Non-Responder) | | | | | | |
|---|---|---|---|---|---|---|
| R: Marker erhöht bei MTX Therapieansprechen (Therapie-Responder Patienten) | | | | | | |
| **Nr** | **Gruppe** | **GeneID** | **Gene Symbol** | **PLAC/MTX p-value** | **PLAC/MTX fold change** | **Therapiesteuerung** |
| 1 | Gruppe 1 | 64753 | CCDC136 | 0.050 | -5.15 | NR |
| 2 | Gruppe 1 | 3281 | HSBP1 | 0.004 | -2.15 | NR |
| 3 | Gruppe 1 | 3485 | IGFBP2 | 0.025 | -1.65 | NR |
| 4 | Gruppe 5 | 10476 | ATP5H | 0.021 | -2.14 | NR |
| 5 | Gruppe 5 | 7812 | CSDE1 | 0.037 | -1.96 | NR |
| 6 | Gruppe 5 | 1485 | CTAG1B | 0.013 | -1.54 | NR |
| 7 | Gruppe 5 | 8320 | EOMES | 0.023 | -2.58 | NR |
| 8 | Gruppe 5 | 10961 | ERP29 | 0.018 | -1.57 | NR |
| 9 | Gruppe 5 | 84893 | FBXO18 | 0.006 | -1.50 | NR |
| 10 | Gruppe 5 | 3059 | HCLS1 | 0.019 | -3.02 | NR |
| 11 | Gruppe 5 | 3182 | HNRNPAB | 0.001 | -2.43 | NR |
| 12 | Gruppe 5 | 3798 | KIF5A | 0.003 | -1.76 | NR |
| 13 | Gruppe 5 | 8079 | MLF2 | 0.042 | -1.58 | NR |
| 14 | Gruppe 5 | 60560 | MAK10 | 0.018 | -1.54 | NR |
| 15 | Gruppe 5 | 8473 | OGT | 0.025 | -1.55 | NR |
| 16 | Gruppe 5 | 5174 | PDZK1 | 0.023 | -2.24 | NR |
| 17 | Gruppe 5 | 23645 | PPP1R15A | 0.007 | -1.82 | NR |
| 18 | Gruppe 5 | 64062 | RBM26 | 0.016 | -2.13 | NR |
| 19 | Gruppe 5 | 23170 | TTLL12 | 0.020 | -2.92 | NR |
| 20 | Gruppe 5 | 91544 | UBXN11 | 0.004 | -1.77 | NR |
| 21 | Gruppe 5 | 6944 | VPS72 | 0.030 | -1.87 | NR |
| 22 | Gruppe 5 | 163033 | ZNF579 | 0.016 | -2.09 | NR |
| 23 | Gruppe 5 | 23625 | FAM89B | 0.013 | -1.70 | NR |
| 24 | Gruppe 5 | 27344 | PCSK1N | 0.005 | -2.99 | NR |
| 25 | Gruppe 5 | 64762 | FAM59A | 0.026 | -3.45 | NR |
| 26 | Gruppe 5 | 30 | ACAA1 | 0.034 | 2.59 | R |
| 27 | Gruppe 5 | 11093 | ADAMTS13 | 0.011 | 1.61 | R |
| 28 | Gruppe 5 | 204 | AK2 | 0.025 | 1.87 | R |
| 29 | Gruppe 5 | 211 | ALAS1 | 0.007 | 3.64 | R |
| 30 | Gruppe 5 | 90416 | C15orf57 | 0.031 | 1.69 | R |
| 31 | Gruppe 5 | 51531 | C9orf156 | 0.008 | 1.69 | R |
| 32 | Gruppe 5 | 1108 | CHD4 | 0.028 | 1.95 | R |
| 33 | Gruppe 5 | 22982 | DIP2C | 0.012 | 1.58 | R |
| 34 | Gruppe 5 | 9454 | HOMER3 | 0.019 | 1.87 | R |
| 35 | Gruppe 5 | 3490 | IGFBP7 | 0.021 | 1. 98 | R |
| 36 | Gruppe 5 | 10445 | MCRS1 | 0.015 | 1.54 | R |
| 37 | Gruppe 5 | 112950 | MED8 | 0.031 | 1.91 | R |
| 38 | Gruppe 5 | 65003 | MRPL11 | 0.003 | 4.15 | R |
| 39 | Gruppe 5 | 7469 | WHSC2 | 0.035 | 1.73 | R |
| 40 | Gruppe 5 | 84081 | CCDC55 | 0.039 | 1.82 | R |
| 41 | Gruppe 5 | 81572 | PDRG1 | 0.013 | 2.16 | R |
| 42 | Gruppe 5 | 5494 | PPM1A | 0.039 | 1.75 | R |
| 43 | Gruppe 5 | 5536 | PPP5C | 0.005 | 1. 98 | R |
| 44 | Gruppe 5 | 9727 | RAB11FIP3 | 0.033 | 1.81 | R |
| 45 | Gruppe 5 | 10111 | RAD50 | 0.012 | 2.55 | R |
| 46 | Gruppe 5 | 6449 | SGTA | 0.004 | 2.49 | R |
| 47 | Gruppe 5 | 116841 | SNAP47 | 0.041 | 1.59 | R |
| 48 | Gruppe 5 | 10290 | SPEG | 0.013 | 2.15 | R |
| 49 | Gruppe 5 | 10422 | UBAC1 | 0.007 | 2.16 | R |
| 50 | Gruppe 5 | 65109 | UPF3B | 0.020 | 2.13 | R |
| 51 | Gruppe 5 | 8882 | ZNF259 | 0.021 | 1.56 | R |

**Tabelle 4: 53 Marker zur Steuerung von anti-TNF Inhibitor Therapie**

| NR: Marker erhöht bei ADA/MTX Kombinationstherapieresistenz (Therapie-Non-Responder) | | | | | | |
|---|---|---|---|---|---|---|
| R: Marker erhöht bei ADA/MTX Kombinationstherapieresistenz (Therapie-Responder Patienten) | | | | | | |
| **Nr** | **Gruppe** | **GeneID** | **Gene Symbol** | **ADA/MTX p**-**value** | **ADA/MTX fold-change** | **ADA/MTX Therapiesteuerung** |
| 52 | Gruppe 6 | 39 | ACAT2 | 0.029 | -1.80 | NR |
| 53 | Gruppe 6 | 348 | APOE | 0.020 | 1.90 | R |
| 54 | Gruppe 6 | 128061 | C1orf131 | 0.013 | 2.16 | R |
| 55 | Gruppe 6 | 79714 | CCDC51 | 0.042 | -2.36 | NR |
| 56 | Gruppe 6 | 6249 | CLIP1 | 0.036 | -1.53 | NR |
| 57 | Gruppe 6 | 30827 | CXXC1 | 0.020 | 1.58 | R |
| 58 | Gruppe 6 | 9704 | DHX34 | 0.010 | -1.77 | NR |
| 59 | Gruppe 6 | 57572 | DOCK6 | 0.011 | -1.69 | NR |
| 60 | Gruppe 6 | 84444 | DOT1L | 0.010 | -2.04 | NR |
| 61 | Gruppe 6 | 23301 | EHBP1 | 0.031 | 1.62 | R |
| 62 | Gruppe 6 | 8661 | EIF3A | 0.009 | 1.71 | R |
| 63 | Gruppe 6 | 1982 | EIF4G2 | 0.039 | -2.20 | NR |
| 64 | Gruppe 6 | 63877 | C10orf84 | 0.020 | -2.01 | NR |
| 65 | Gruppe 6 | 54865 | GPATCH4 | 0.003 | 2.36 | R |
| 66 | Gruppe 6 | 2922 | GRP | 0.039 | -1.80 | NR |
| 67 | Gruppe 6 | 84064 | HDHD2 | 0.010 | -1.65 | NR |
| 68 | Gruppe 6 | 80895 | ILKAP | 0.018 | 1.61 | R |
| 69 | Gruppe 6 | 23479 | ISCU | 0.040 | 1.58 | R |
| 70 | Gruppe 6 | 57498 | KIDINS220 | 0.011 | -1.72 | NR |
| 71 | Gruppe 6 | 3875 | KRT18 | 0.018 | -1.63 | NR |
| 72 | Gruppe 6 | 29094 | HSPC159 | 0.011 | -1.94 | NR |
| 73 | Gruppe 6 | 79888 | LPCAT1 | 0.025 | -2.10 | NR |
| 74 | Gruppe 6 | 4357 | MPST | 0.002 | 2.94 | R |
| 75 | Gruppe 6 | 10528 | NOP56 | 0.034 | 1.50 | R |
| 76 | Gruppe 6 | 51602 | NOP58 | 0.000 | 2.21 | R |
| 77 | Gruppe 6 | 9315 | C5orf13 | 0.004 | 1. 60 | R |
| 78 | Gruppe 6 | 23762 | OSBP2 | 0.021 | -1.87 | NR |
| 79 | Gruppe 6 | 55593 | OTUD5 | 0.021 | -1.74 | NR |
| 80 | Gruppe 6 | 79668 | PARP8 | 0.015 | -1.95 | NR |
| 81 | Gruppe 6 | 23089 | PEG10 | 0.016 | 2.09 | R |
| 82 | Gruppe 6 | 9360 | PPIG | 0.035 | 1.66 | R |
| 83 | Gruppe 6 | 84687 | PPP1R9B | 0.044 | 1.73 | R |
| 84 | Gruppe 6 | 5589 | PRKCSH | 0.005 | -2.54 | NR |
| 85 | Gruppe 6 | 5834 | PYGB | 0.021 | -1.57 | NR |
| 86 | Gruppe 6 | 117584 | RFFL | 0.014 | -1.94 | NR |
| 87 | Gruppe 6 | 9025 | RNF8 | 0.032 | -1.54 | NR |
| 88 | Gruppe 6 | 6091 | ROBO1 | 0.005 | 2.14 | R |
| 89 | Gruppe 6 | 6240 | RRM1 | 0.023 | -1.68 | NR |
| 90 | Gruppe 6 | 22937 | SCAP | 0.041 | -1.64 | NR |
| 91 | Gruppe 6 | 23256 | SCFD1 | 0.005 | 2.39 | R |
| 92 | Gruppe 6 | 6382 | SDC1 | 0.007 | -2.15 | NR |
| 93 | Gruppe 6 | 10483 | SEC23B | 0.018 | -2.13 | NR |
| 94 | Gruppe 6 | 6576 | SLC25A1 | 0.031 | -3.58 | NR |
| 95 | Gruppe 6 | 92521 | CYTSB | 0.017 | -2.91 | NR |
| 96 | Gruppe 6 | 23635 | SSBP2 | 0.021 | -2.44 | NR |
| 97 | Gruppe 6 | 6612 | SUMO3 | 0.014 | -1.62 | NR |
| 98 | Gruppe 6 | 84260 | TCHP | 0.026 | -1.68 | NR |
| 99 | Gruppe 6 | 9804 | TOMM20 | 0.021 | 1.97 | R |
| 100 | Gruppe 6 | 55850 | USE1 | 0.046 | -1.64 | NR |
| 101 | Gruppe 6 | 89891 | WDR34 | 0.019 | -1.71 | NR |
| 102 | Gruppe 6 | 54521 | WDR44 | 0.007 | 2.18 | R |
| 103 | Gruppe 6 | 23036 | ZNF292 | 0.003 | 2.85 | R |
| 104 | Gruppe 6 | 6234 | RPS28 | 0.028 | -1.55 | NR |

### Beispiele

### Beispiel 1: Modellbildung zur Diskrimination von Therapie-Respondern und -Non-Respondern mittels PLS

Auf der Basis der EULAR / ACR Leitlinien für die Behandlung von RA Patienten, ist das oberste Ziel mittels einer geeigneten Therapie eine Remission der RA zu erreichen. Diese wird häufig als Erreichen eines DAS28 Werts von <2.6 definiert. Falls dies nicht möglich ist, sollte zumindest eine möglich niedrige Krankheitsaktivität erreicht werden.

In einem ersten Schritt wurde ein Partial Least Squares (PLS) Regressionsmodell erstellt. Mittels eines sogenannten PLS Scoreplots ist es möglich zu visualisieren, ob es unter Verwendung der ausgewählten Antigene möglich ist, Patienten in sogenannte Therapieresponder und Non-Responder zu diskriminieren. Für die Modellbildung wurden zunächst alle Antigene aus Tabelle 1 verwendet und die Autoantikörperspiegel aller Serumproben zum Zeitpunkt T0 vor Therapiebeginn analysiert.

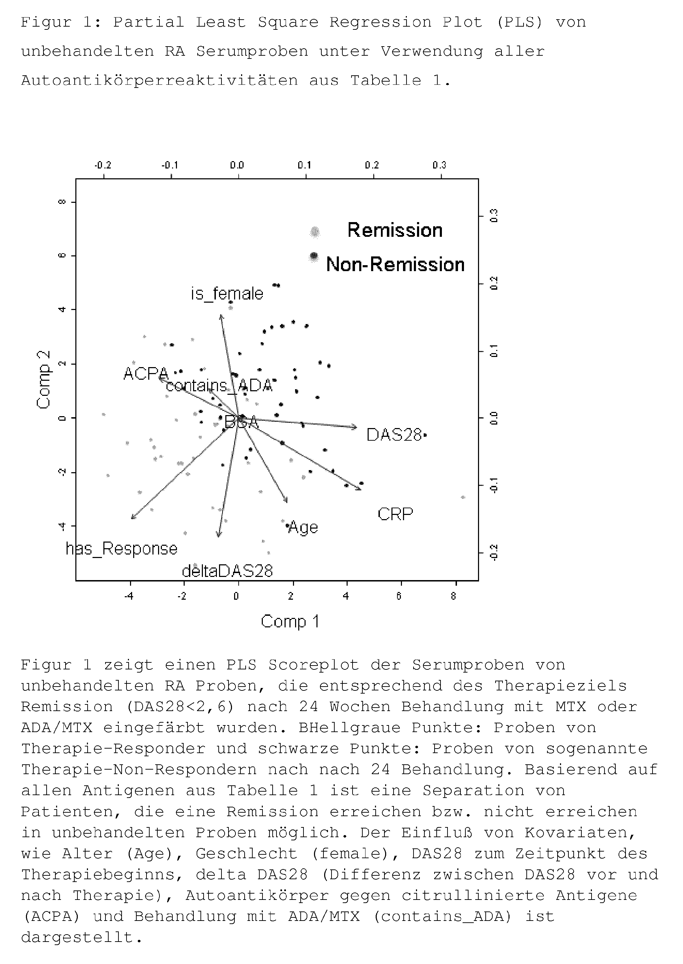

Figur 1 zeigt einen PLS-Scoreplot für alle Antigene aus Tabelle 1 und deren Autoantikörpermesswerte. Figur 1 zeigt, dass die Separation von Patienten, die das Therapieziel Remission erreichen bzw. verfehlen vor Therapiebeginn prinzipiell möglich ist. Somit können aus Tabelle 1 Antigene spezifiziert werden, um verschiedene Therapiestellungen zu begleiten.

### Beispiel 2: Kalkulation von Antigen Panels zur Vorhersage des Ansprechens auf MTX Monotherapie

Aufgrund der klinischen und serologischen Heterogenität der RA Erkrankung ist es nicht möglich, mit nur einem Biomarker ein Ansprechen einer MTX Therapie für alle RA Patienten vorauszusagen. Daher ist es erforderlich, möglichst unkorrelierte Autoantigene zu sogenannten Biomarker Panels zu kombinieren.

Gruppe 5 der Antigene in Tabelle 3 umfasst die wichtigsten 51 Antigene, die für die Berechnung von Biomarker Panels zur MTX Therapiesteuerung verwendet werden.

Gruppe 5 enthält 26 Antigene 26 Autoantikörperreaktivitäten (Antigene), die in RA Patienten messbar sind, die auf eine MTX Basistherapie ansprechen: ACAA1, ADAMTS13, AK2, ALAS1, C15orf57, C9orf156, CHD4, DIP2C, HOMER3, IGFBP7, MCRS1, MED8, MRPL11, WHSC2, CCDC55, PDRG1, PPM1A, PPP5C, RAB11FIP3, RAD50, SGTA, SNAP47, SPEG, UBAC1, UPF3B, ZNF259. Aus diesen 26 Antigenen können Antigene ausgewählt werden, die sich besonders gut als Marker in kleineren Panels ergänzen. Panel 1 in Tabelle 5 enthält eine Kombination von 11 Markern, die gezielt dazu verwendet werden können, um Patienten zu identifizieren, die voraussichtlich auf MTX Therapie ansprechen werden.

Für die Generierung von Panels wurden basierend auf den univariaten Ergebnisse Antigene ausgewählt, die einen p-Wert für den nichtparametrischen Mittelwertvergleich der Serumproben vor Therapiebeginn zwischen RA Patienten, die nach 24 Wochen Behandlung mit MTX auf die Therapie ansprechen bzw. nicht ansprechen. Es wurde ein p-Wert von <0,05 und gleichzeitig eine unterschiedliche Medianverteilung von 1,5 zwischen den beiden Gruppen zugrunde gelegt. Für die in Tabelle 3 genannten 52 Antigene wurde im Rahmen einer genesteten Kreuzvalidierung ein L1-penalisiertes logistisches Regressionsmodell aufgestellt. Antigene, die im Rahmen der Modellbildung nicht berücksichtigt wurden, wurden aus der weiteren Betrachtung entfernt.

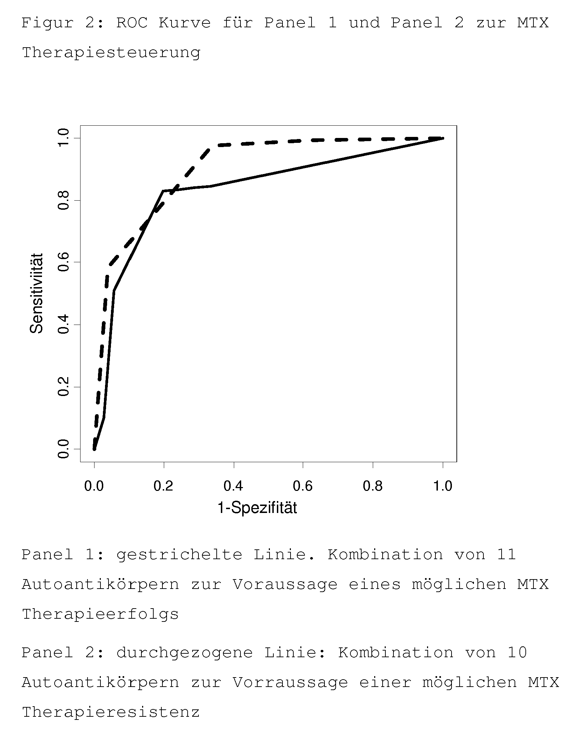

Wie in Tabelle 5 zusammengefasst wurde mit 11 Markern des Panels 1 eine AUC von 0.9 bei einer mittleren Sensitivität von 98% und einer mittleren Spezifität 72% erreicht. Figur 2 zeigt die ROC Kurve für Panel 1.

### Bespiel 3: Kalkulation von Antigen Panels zur Vorhersage einer MTX Therapieresistenz

Gruppe 5 in Tabelle 3 enthält 26 Autoantikörperreaktivitäten (Antigene), die in RA Patienten messbar sind, die vorrausichtlich nicht auf MTX Therapie ansprechen werden: CCDC136, HSBP1, IGFBP2, ATP5H, CSDE1, CTAG1B, EOMES, ERP29, FBXO18, HCLS1, HNRNPAB, KIF5A, MLF2, MAK10, OGT, PDZK1, PPP1R15A, RBM26, TTLL12, UBXN11, VPS72, ZNF579, FAM89B, PCSK1N, FAM59A.

Aus diesen 26 Antigenen können Antigene ausgewählt, die sich besonders gut als Marker in kleineren Panels ergänzen. Panel 1 in Tabelle 5 enthält eine Kombination von 10 Markern, die gezielt dazu verwendet werden können, um Patienten zu identifizieren, die voraussichtlich nicht auf MTX Therapie ansprechen werden.

Tabelle 5 zeigt unterschiedliche Kombinationen von Antigenen, die für die Berechnung der Biomarker Panels für die MTX Therapiesteuerung verwendet werden können.

**Tabelle 5: Antigene und Biomarkerpanels für die MTX Therapiesteuerung**

| **Marker Nr** | **Antigen Symbol** | **Panel** | **Therapiesteuerung** | **AUC** | **Sens.** | **Spec.** |
|---|---|---|---|---|---|---|
| 43 | PPP5C | 1 | MTX Therapieansprechen | 0.9 | 0.89 | 0.72 |
| 38 | MRPL11 | | | | | |
| 31 | TRMO | | | | | |
| 45 | RAD50 | | | | | |
| 27 | ADAMTS13 | | | | | |
| 33 | DIP2C | | | | | |
| 32 | CHD4 | | | | | |
| 29 | ALAS1 | | | | | |
| 51 | ZPR1 | | | | | |
| 41 | PDRG1 | | | | | |
| 37 | MED8 | | | | | |
| 11 | HNRNPAB | 2 | MTX Therapieresistenz | 0.88 | 0.82 | 0.9 |
| 18 | RBM26 | | | | | |
| 20 | UBXN11 | | | | | |
| 23 | FAM89B | | | | | |
| 19 | TTLL12 | | | | | |
| 17 | PPP1R15A | | | | | |
| 25 | GAREM | | | | | |
| 12 | KIF5A | | | | | |
| 10 | HCLS1 | | | | | |
| 22 | ZNF579 | | | | | |

Für das Panel 2 wurde beispielhaft gezeigt, dass sogenannte MTX Therapieversager mit einer mittleren AUC von 0.88 und einer Sensitivität von 82% bei einer Spezifität von 90% identifiziert werden können.

Figur 2 zeigt die Sensitivität und Spezifität, sowie die Fläche unter der Kurve (Area under the Curve, AUC) für die Panel 1 und Panel 2.

### Beispiel 4: Kalkulation von Antigen Panels zur Vorhersage des Therapieerfolgs nach Behandlung mit anti-TNF Inihibitoren.

Gruppe 6 in Tabelle 4 enthält 20 Autoantikörperreaktivitäten (Antigene), die in RA Patienten messbar sind, die vorrausichtlich auf eine anti-TNF alpha Inhibitor Therapie ansprechen werden: APOE, C1orf131, CXXC1, EHBP1, EIF3A, GPATCH4, ILKAP, ISCU, MPST, NOP56, NOP58, C5orf13, PEG10, PPIG, PPP1R9B, ROBO1, SCFD1, TOMM20, WDR44, ZNF292

Aus der Gruppe 6 können 20 Antigene ausgewählt werden, die sich besonders gut als Marker in kleineren Panels ergänzen. Panel 3 in Tabelle 5 enthält eine Kombination von 11 Markern, die gezielt dazu verwendet werden können, um Patienten zu identifizieren, die voraussichtlich nicht MTX Therapie ansprechen werden.

Tabelle 6 zeigt unterschiedliche Kombinationen von Antigenen, die für die Berechnung der Biomarker Panels für die ADA/MTX Therapiesteuerung verwendet werden können.

**Tabelle 6: Antigene und Biomarkerpanels für die ADA/MTX Therapiesteuerung**

| **Marker Nr** | **Antigen Symbol** | **Panel** | **Therapiesteuerung** | **AUC** | **Sens.** | **Spec.** |
|---|---|---|---|---|---|---|
| 76 | NOP58 | 3 | ADA/MTX Therapieansprechen | 0.82 | 0.79 | 0.78 |
| 91 | SCFD1 | | | | | |
| 74 | MPST | | | | | |
| 68 | ILKAP | | | | | |
| 88 | ROBO1 | | | | | |
| 69 | ISCU | | | | | |
| 61 | EHBP1 | | | | | |
| 83 | PPP1R9B | | | | | |
| 103 | ZNF292 | | | | | |
| 99 | TOMM20 | | | | | |
| 54 | C1orf131 | | | | | |
| 95 | SPECC1L | 4 | ADA/MTX Therapieresistenz | 0.95 | 0.88 | 0.89 |
| 93 | SEC23B | | | | | |
| 67 | HDHD2 | | | | | |
| 56 | CLIP1 | | | | | |
| 101 | WDR34 | | | | | |
| 63 | EIF4G2 | | | | | |
| 96 | SSBP2 | | | | | |
| 90 | SCAP | | | | | |
| 85 | PYGB | | | | | |
| 59 | DOCK6 | | | | | |
| 86 | RFFL | | | | | |
| 97 | SUMO3 | | | | | |

Für das Panel 3 wurde beispielhaft gezeigt, dass Patienten, die wahrscheinlich auf eine ADA/MTX Kombinationstherapie ansprechen werden mit einer mittleren AUC von 0.82 und einer Sensitivität von 79% bei einer Spezifität von 78% identifiziert werden können.

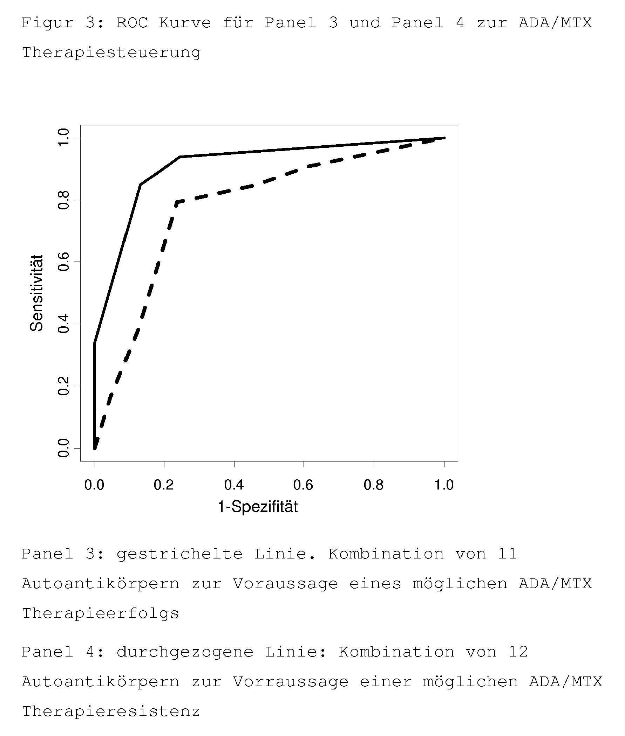

Figur 3 zeigt die Sensitivität und Spezifität, sowie die Fläche unter der Kurve (Area under the Curve, AUC) für die Panel 3 und Panel 4.

### Beispiel 5: Kalkulation von Antigen Panels zur Vorhersage eines Therapieversagens nach Behandlung mit anti-TNF Inihibitoren.

Gruppe 6 in Tabelle 4 enthält 33 Autoantikörperreaktivitäten (Antigene), die in RA Patienten messbar sind, die vorrausichtlich nicht auf eine anti-TNF alpha Inhibitor Therapie ansprechen werden: ACAT2, CCDC51, CLIP1, DHX34, DOCK6, DOT1L, EIF4G2, C10orf84, GRP, HDHD2, KIDINS220, KRT18, HSPC159, LPCAT1, OSBP2, OTUD5, PARP8, PRKCSH, PYGB, RFFL, RNF8, RRM1, SCAP, SDC1, SEC23B, SLC25A1, CYTSB, SSBP2, SUMO3, TCHP, USE1, WDR34, RPS28

Aus der Gruppe 6 können 33 Antigene ausgewählt werden, die sich besonders gut als Marker in kleineren Panels ergänzen. Panel 4 in Tabelle 6 enthält eine Kombination von 12 Markern, die gezielt dazu verwendet werden können, um Patienten zu identifizieren, die voraussichtlich nicht auf eine ADA/MTX Therapie ansprechen werden und daher mit einem alternativen biologischen DMARD behandelt werden sollten.

Für das Panel 4 wurde beispielhaft gezeigt, dass sogenannte ADA/MTX Therapieversager mit einer mittleren AUC von 0.95 und einer Sensitivität von 88% bei einer Spezifität von 89% identifiziert werden können.

Figur 3 zeigt die Sensitivität und Spezifität, sowie die Fläche unter der Kurve (Area under the Curve, AUC) für die Panel 4.

### Literatur:

Aletaha, D., T. Neogi, et al. (2010). "2010 Rheumatoid arthritis classification criteria: an American College of Rheumatology/European League Against Rheumatism collaborative initiative." Ann Rheum Dis 69(9): 1580-1588.
Detert, J., H. Bastian, et al. (2013). "Induction therapy with adalimumab plus methotrexate for 24 weeks followed by methotrexate monotherapy up to week 48 versus methotrexate therapy alone for DMARD-naive patients with early rheumatoid arthritis: HIT HARD, an investigator-initiated study." Ann Rheum Dis 72(6): 844-850.
Hueber, W., B. Tomooka, et al. (2009). "Blood autoantibody and cytokine profiles predict response to anti-tumor necrosis factor therapy in rheumatoid arthritis." Arthritis Res Ther 11(3): R76.
Smolen, J. S., R. Landewé, et al. (2010). "EULAR recommendations for the management of rheumatoid arthritis with synthetic and biological disease-modifying antirheumatic drugs." Ann Rheum Dis 69(6): 964-975.
Somers, K., P. Geusens, et al. (2011). "Novel autoantibody markers for early and seronegative rheumatoid arthritis." J Autoimmun 36(1): 33-46.

## Patentansprüche

1. Verfahren zur Therapiesteuerung von Patienten mit rheumatoider Arthritis in medikamentöser Behandlung, wobei mittels einer in-vitro Diagnose Responder und / oder Non-Responder auf die medikamentöse Behandlung identifiziert werden, **dadurch gekennzeichnet, dass** mindestens eine Markersequenz aus der Gruppe SEQ ID No. 1-208 ausgewählt ist.

2. Verfahren zur Therapiesteuerung von Patienten mit rheumatoider Arthritis in medikamentöser Behandlung nach Anspruch 1, wobei mittels einer in-vitro Diagnose therapieresistente Patienten auf die medikamentöse Behandlung identifiziert werden, **dadurch gekennzeichnet, dass** mindestens eine Markersequenz aus der Gruppe SEQ ID No. 1-3 und/oder 105-107, SEQ ID No. 4-51 und/oder 108-155, SEQ ID No. 52-104 und/oder 156-208 ausgewählt ist.

3. Verfahren zur Therapiesteuerung von Patienten mit rheumatoider Arthritis in medikamentöser Behandlung nach Anspruch 1 oder Anspruch 2, wobei die medikamentöse Behandlung die Arzneimittel Methotrexate, Azathioprin, Sulfasalazin, Chloroquin/Hydroxychloroquin, Leflunomid, Cyclophosphamid, D-Penicillamin oder Ciclosporin umfassen, **dadurch gekennzeichnet, dass** mindestens eine Markersequenz aus der Gruppe SEQ ID No. 1-3 und/oder 105-107, SEQ ID No. 4-51 und/oder 108-155 ausgewählt ist.

4. Verfahren zur Therapiesteuerung von Patienten mit rheumatoider Arthritis in medikamentöser Behandlung nach Anspruch 3, wobei die medikamentöse Behandlung das Arzneimittel Methotrexate umfasst.

5. Verfahren zur Therapiesteuerung von Patienten mit rheumatoider Arthritis in medikamentöser Behandlung nach einem der vorstehenden Patentansprüche, wobei die medikamentöse Behandlung die Arzneimittel monoklonale therapeutische Proteine, TNF-Blocker, anti-TNF-Blocker umfassen, **dadurch gekennzeichnet, dass** mindestens eine Markersequenz aus der Gruppe SEQ ID No. 52-104 und/oder 156-208 ausgewählt ist.

6. Verfahren zur Therapiesteuerung von Patienten mit rheumatoider Arthritis in medikamentöser Behandlung nach Anspruch 5, **dadurch gekennzeichnet, dass** die Behandlung in Kombination mit Arzneimitteln ausgewählt aus der Gruppe Methotrexate, Azathioprin, Sulfasalazin, Chloroquin/Hydroxychloroquin, Leflunomid, Cyclophosphamid, D-Penicillamin oder Ciclosporin erfolgt.

7. Verfahren zur Therapiesteuerung von Patienten mit rheumatoider Arthritis in medikamentöser Behandlung nach Anspruch 6, wobei die medikamentöse Behandlung das Arzneimittel Methotrexate umfasst.

8. Verfahren zur Therapiesteuerung von Patienten mit rheumatoider Arthritis in medikamentöser Behandlung nach einem der vorstehenden Ansprüchen, umfassend weitere klinische Entscheidungen, wie Änderung der Therapie, Abbruch der Therapie, Wechsel des Medikaments, Hospitalisierung.

9. Verfahren zur Therapiesteuerung von Patienten mit rheumatoider Arthritis in medikamentöser Behandlung nach enem der vorstehenden Ansprüche, wobei mittels einer in-vitro Diagnose Responder und / oder Non-Responder auf die medikamentöse Behandlung identifiziert werden, **dadurch gekennzeichnet, dass** bis zu zwölf Markersequenzen aus der Gruppe SEQ ID No. 1-208 ausgewählt sind.

10. Verfahren zur Therapiesteuerung von Patienten mit rheumatoider Arthritis in medikamentöser Behandlung nach enem der vorstehenden Ansprüche, **dadurch gekennzeichnet, dass** eine Remission der rheumatoiden Arthritis erfolgt.

11. Verfahren zur Therapiesteuerung von Patienten mit rheumatoider Arthritis in medikamentöser Behandlung nach Anspruch 9, wobei die Markersequenzen ausgewählt sind aus der Gruppe
PPP5C (SEQ ID No. 43, 147), MRPL11 (SEQ ID No. 38, 142), TRMO (SEQ ID No. 31, 135), RAD50 (SEQ ID No. 45, 149), ADAMTS13 (SEQ ID No. 27, 131), DIP2C (SEQ ID No. 33, 137), CHD4 (SEQ ID No. 32, 136), ALAS1 (SEQ ID No. 29, 133), ZPR1 (SEQ ID No. 51, 155), PDRG1 (SEQ ID No. 41, 145) oder
MED8 (SEQ ID No. 37, 141), HNRNPAB (SEQ ID No. 11, 115), RBM26 (SEQ ID No. 18, 122), UBXN11 (SEQ ID No. 20, 124), FAM89B (SEQ ID No. 23, 127), TTLL12 (SEQ ID No. 19, 123), PPP1R15A (SEQ ID No. 17, 121), GAREM (SEQ ID No. 25, 129), KIF5A (SEQ ID No. 12, 116), HCLS1 (SEQ ID No. 10, 114), ZNF579 (SEQ ID No. 22, 126),
oder
NOP58 (SEQ ID No. 76, 180), SCFD1 (SEQ ID No. 91, 195), MPST (SEQ ID No. 74, 178), ILKAP (SEQ ID No. 68, 172),
ROBO1 (SEQ ID No. 88, 192), ISCU (SEQ ID No. 69, 173), EHBP1 (SEQ ID No. 61, 165), PPP1R9B (SEQ ID No. 83, 187), ZNF292 (SEQ ID No. 103, 207), TOMM20 (SEQ ID No. 99, 203), C1orf131 (SEQ ID No. 54, 158),
oder
SPECC1L (SEQ ID No. 95, 199), SEC23B (SEQ ID No. 93, 197), HDHD2 (SEQ ID No. 67, 171, CLIP1 (SEQ ID No. 56,160), WDR34(SEQ ID No. 101, 205), EIF4G2 (SEQ ID No. 63, 167), SSBP2(SEQ ID No. 96, 200), SCAP (SEQ ID No. 90, 194), PYGB (SEQ ID No. 85, 189), DOCK6 (SEQ ID No. 59, 163), RFFL (SEQ ID No. 86, 190), SUMO3 (SEQ ID No. 97, 201).

12. Panel enthaltend mindestens fünf Markersequenzen ausgewählt aus der Gruppe
PPP5C (SEQ ID No. 43, 147), MRPL11 (SEQ ID No. 38, 142), TRMO (SEQ ID No. 31, 135), RAD50 (SEQ ID No. 45, 149), ADAMTS13 (SEQ ID No. 27, 131), DIP2C (SEQ ID No. 33, 137), CHD4 (SEQ ID No. 32, 136), ALAS1 (SEQ ID No. 29, 133), ZPR1 (SEQ ID No. 51, 155), PDRG1 (SEQ ID No. 41, 145) oder
MED8 (SEQ ID No. 37, 141), HNRNPAB (SEQ ID No. 11, 115), RBM26 (SEQ ID No. 18, 122), UBXN11 (SEQ ID No. 20, 124), FAM89B (SEQ ID No. 23, 127), TTLL12 (SEQ ID No. 19, 123), PPP1R15A (SEQ ID No. 17, 121), GAREM (SEQ ID No. 25, 129), KIF5A (SEQ ID No. 12, 116), HCLS1 (SEQ ID No. 10, 114), ZNF579 (SEQ ID No. 22, 126),
oder
NOP58 (SEQ ID No. 76, 180), SCFD1 (SEQ ID No. 91, 195), MPST (SEQ ID No. 74, 178), ILKAP (SEQ ID No. 68, 172), ROBO1 (SEQ ID No. 88, 192), ISCU (SEQ ID No. 69, 173), EHBP1 (SEQ ID No. 61, 165), PPP1R9B (SEQ ID No. 83, 187), ZNF292 (SEQ ID No. 103, 207), TOMM20 (SEQ ID No. 99, 203), C1orf131 (SEQ ID No. 54, 158),
oder
SPECC1L (SEQ ID No. 95, 199), SEC23B (SEQ ID No. 93, 197), HDHD2 (SEQ ID No. 67, 171, CLIP1 (SEQ ID No. 56,160), WDR34(SEQ ID No. 101, 205), EIF4G2 (SEQ ID No. 63, 167), SSBP2(SEQ ID No. 96, 200), SCAP (SEQ ID No. 90, 194), PYGB (SEQ ID No. 85, 189), DOCK6 (SEQ ID No. 59, 163), RFFL (SEQ ID No. 86, 190), SUMO3 (SEQ ID No. 97, 201).
